# FASCICULE DE BREVET EUROPEEN

(11) **EP 2 699 227 B1**
(45) Date de publication et mention de la délivrance du brevet: **12.07.2017**
(21) Numéro de dépôt: 12722441.8
(22) Date de dépôt: 19.04.2012
(51) Int. Cl.: A61K 36/185, A61K 36/30, A61K 36/66, A61K 8/97, A61Q 19/08

(54) **COMPLEXE D'EXTRAITS VÉGÉTAUX POUR LA PROTECTION DE LA PEAU.**
PFLANZENEXTRAKTKOMPLEX ZUM SCHUTZ DER HAUT
PLANT EXTRACT COMPLEX FOR SKIN PROTECTION

(30) Priorité: 20.04.2011 FR 1153426
(43) Date de publication de la demande: 26.02.2014
(73) Titulaire: SOCIETE DE RECHERCHE COSMETIQUE SARL, 2314 Luxembourg (LU)
(72) Inventeur: LECONTE, Nadine, F-92600 Asnieres Sur Seine (FR); LECLERE, Jacques, F-45500 Saint-Gondon (FR)
(74) Mandataire: Goulard, Sophie
(86) Numéro de dépôt international: PCT/FR2012/050857
(87) Numéro de publication internationale: WO 2012/172218

(56) Documents cités:
- FR-A1- 2 871 382
- FR-A1- 2 931 070
- US-A1- 2007 134 189

## Description

La présente invention concerne une nouvelle composition cosmétique et/ou dermatologique utile pour la protection de la peau, et plus particulièrement une composition à base d'extraits d'anchuse, de pétales de coquelicot et de passiflore destinée à traiter et/ou à prévenir les signes du vieillissement cutané.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'épiderme est principalement composé de kératinocytes (90% des cellules épidermiques) qui synthétisent la kératine, de mélanocytes (2 à 3% des cellules épidermiques) responsables de la pigmentation de la peau, et des cellules de Langerhans qui jouent un rôle immunologique. L'épiderme, dont l'épaisseur est variable selon les différentes parties du corps, constitue la couche externe de la peau et par conséquent il joue un rôle fondamental pour assurer la protection et le maintien d'une bonne trophicité. C'est pourquoi de nombreuses compositions ont été mises au point afin de le protéger et d'améliorer ses fonctions, et notamment de renforcer son élasticité et sa fermeté.

Le derme est la couche la plus épaisse, riche en nerfs, en vaisseaux sanguins et en glandes sudoripares, et se compose principalement de collagène, d'élastine et de protéoglycanes. Ces trois types de molécules sont synthétisés par les fibroblastes dermiques. Les fibres de collagène, qui représentent 70% du poids sec du derme, assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau peut être intrinsèque, ou extrinsèque c'est-à-dire provoqué par l'environnement, y compris les agressions climatiques, qui peuvent notamment contribuer à accélérer la dégradation du collagène du derme, et en particulier l'exposition au soleil, les variations de températures et les radicaux libres. Les premiers signes du vieillissement de la peau, tels que les rides et ridules, sont généralement provoqués par le stress et les changements biologiques et physiologiques, accélérés par l'environnement extérieur ou par les modes de vie. L'apparition de marques pigmentaires, la diminution de l'épaisseur de la peau et son affaissement sont également des changements observés au cours du vieillissement. A l'échelle de la peau, le vieillissement provoque notamment une diminution des synthèses protéiques (collagène, élastine), une diminution de la synthèse des protéoglycanes, ainsi qu'une élévation des métalloprotéinases de type MMP3. La capacité de la peau à remplacer le collagène endommagé diminue non seulement avec le temps mais aussi avec des facteurs environnementaux et avec l'état de stress, et des espaces et des irrégularités apparaissent progressivement dans le réseau du collagène.

Le vieillissement de la peau s'accompagne souvent de sensations de pesanteur, de pression durant le sommeil et aussi de contractions des muscles faciaux, déterminant des rides d'expression, notamment autour des yeux et de la bouche, ou la modification de microrelief épidermique, notamment en raison du stress.

On a proposé d'utiliser des compositions cosmétiques contenant des substances à action exfoliante comme les alpha-hydroxy acides, par exemple l'acide lactique et l'acide glycolique, pour faciliter l'élimination des cellules mortes et stimuler la formation de collagène et d'élastine. On connaît aussi des compositions à base d'acide rétinoïque ou de rétinol qui peuvent agir contre les signes du vieillissement cutané tels que la sécheresse, la perte d'élasticité de la peau et la formation de rides.

On a aussi proposé d'incorporer des substances d'origine végétale dans des compositions destinées au traitement des symptômes du vieillissement de la peau. Ainsi, le brevet FR-A-2.761.607 décrit une composition comportant un dérivé de silanol méthylé et un dérivé d'une protéine végétale hydrolysée, additionnée le cas échéant d'un dérivé de vitamine C. Le brevet FR-A-2.783.169 décrit l'utilisation de penta-peptides du type Lys-Thr-Thr-Lys-Ser dans des compositions topiques pour favoriser la synthèse du collagène et des glycosaminoglycannes, et par conséquent la régénération cutanée. Un exemple de pentapeptide disponible dans le commerce est le Matrixyl^{®}. La demande WO 2004062637 décrit une composition cosmétique et/ou dermatologique à base d'inhibiteurs de métallo-protéinases matricielles, en particulier d'extrait de Siegesbeckia, et de lipopeptides permettant le traitement et la prévention des signes du vieillissement cutané tels que l'apparition de rides et la perte d'élasticité de la peau. Une composition à base d'extrait de graines de mimosa, permettant d'agir contre l'apparition des rides et la perte d'élasticité de la peau, est décrite dans la demande WO 2007144518.

La passiflore, de la famille des passifloraceae, est une plante médicinale vivace utilisée en infusions pour son effet calmant et sédatif. Elle comprend plusieurs centaines d'espèces, dont certaines, telles que Passiflora edulis et Passiflora ligularis, sont connues pour donner des fruits comestibles (fruit de la passion et grenadelle). Le brevet EP 1.541.037 décrit des compositions pour administration par voie orale, contenant un extrait de passiflore et des acides gras, pouvant être utilisées comme additifs alimentaires. La demande de brevet EP 1.537.789 décrit une composition à activité anti-inflammatoire pour application topique ou pour administration par voie orale contenant un caroténoïde, du tocophérol, un extrait de passiflore et un extrait de myrtille ; de telles compositions sont présentées comme efficaces pour lutter contre les effets préjudiciables engendrés par une exposition excessive aux radiations solaires. Des compositions cosmétiques destinées à lutter contre le vieillissement de la peau et contenant des huiles riches en acides gras essentiels ainsi qu'un extrait de passiflore sont décrites dans le brevet EP 1.002.524. La demande de brevet US 2007/134189 décrit des compositions antirides à base d'extraits de passiflore, de menthe, de coquelicot et de myrte. La demande de brevet JP 2002-332224 décrit une composition cosmétique pour lutter contre le vieillissement cutané contenant un extrait de passiflore à titre de composé permettant d'éliminer les ions superoxyde et un additif choisi parmi les agents hydratants, les antioxydants, les activateurs de cellules, les agents de blanchiment et les filtres solaires.

Des extraits d'anchuse (Anchusa officinalis) ont été proposés dans des compositions alimentaires, en association avec d'autres extraits végétaux tels que Magnolia officinalis, Nelumbo nucifera et Origanum vulgare, pour inhiber la formation de mercaptans sous l'action de bactéries, comme indiqué dans le brevet JP 2005-162697. Des études ont montré que des extraits de racines d'Anchusa strigosa peuvent avoir un effet protecteur de l'ulcère induit par l'éthanol chez le rat (AM Disi et al., J. Ethnopharmacol. 1998, p.189-98). L'utilisation d'extraits d'anchuse à titre d'agent hydratant ou adoucissant dans des compositions cosmétiques a déjà été proposée, notamment dans la demande de brevet US 2006/018867. L'association de passiflore et d'anchuse a été décrite dans la demande WO 2009147345, ainsi que son utilisation pour lutter contre les contractions musculaires, en particulier les contractions faciales et les rides d'expression.

Le coquelicot (Papaver rhoeas) contient de la rhoeadine, alcaloïde de la série des tétrahydrobenzazépines, qui est supposée avoir des propriétés neuroleptiques. Cette plante a été décrite comme pouvant être utilisée dans des cas d'éréthisme cardiaque de l'adulte, comme calmant chez les adultes et les enfants, et aussi pour le traitement symptomatique de la toux.

Des compositions comprenant des extraits de coquelicot (Papaver rhoeas) en association avec des extraits de lotus bleu (Nymphaea caerulea), présentant des effets d'inhibition des contractions musculaires faciales, sont décrites dans le brevet FR 2.871.382. Ces extraits sont obtenus à partir des graines des plantes. Une composition pharmaceutique administrable par voie orale à base d'extraits de pétales de coquelicot a été proposée dans le brevet GB 171920 pour le traitement de la tuberculose. La demande WO 2009101301 décrit l'utilisation des pétales de coquelicot (Papaver rhoeas) pour la préparation de compositions topiques favorisant la nutrition des cellules dermiques et épidermiques. Une étude pharmaco-toxicologique chez la souris a montré que des extraits de pétales de coquelicot pouvaient avoir un effet sédatif à des doses importantes (R. Soulimari et al., "Medicinal and Aromatic Plants Abstracts", vol. 23 n° 5, oct. 2001).

Cependant, bien que de nombreuses compositions cosmétiques et dermatologiques aient été proposées, il existe toujours un besoin de pouvoir disposer de nouvelles compositions topiques alternatives permettant de protége la peau et de lutter contre les signes du vieillissement cutané, et plus particulièrement des compositions topiques à base d'extraits végétaux appropriés provenant de plantes connues pour leurs propriétés favorables à une telle activité.

Les études effectuées par la demanderesse ont montré que l'association des plantes constituées par l'anchuse, le coquelicot et la passiflore procure des effets inattendus utiles pour la protection de la peau et dans la prévention et le traitement des signes du vieillissement cutané.

La présente invention a donc pour objet une nouvelle composition cosmétique et/ou dermatologique pour application topique à base d'extraits de plantes, et plus particulièrement à base d'extraits d'anchuse, de pétales ou de mucilage de pétales de coquelicot et de passiflore, en association.

Les compositions de l'invention sont des compositions topiques qui comprennent une quantité efficace d'extraits d'anchuse, de pétales ou de mucilage de pétales de coquelicot et de passiflore, en association, pour assurer une bonne protection de la peau.

De telles compositions présentent d'excellentes propriétés utilisables en cosmétique et en dermatologie pour les soins de la peau, plus particulièrement pour protéger la peau des agressions de l'environnement et pour prévenir et/ou traiter les signes du vieillissement cutané.

L'invention a également pour objet une composition pharmaceutique à base d'extraits d'anchuse, de pétales ou de mucilage de pétales de coquelicot et de passiflore, en association, procurant des effets sur la protection et la régénération de l'ADN mitochondrial, pour une utilisation dans la prévention et/ou le traitement des signes du vieillissement cutané.

Les compositions suivant la présente invention se distinguent en ce qu'elles comprennent une association d'extraits d'anchuse, de pétales ou de mucilage de pétales de coquelicot et de passiflore en une quantité efficace pour procurer un effet sur la protection et la régénération de l'ADN mitochondrial, permettant la prévention et/ou le traitement des signes du vieillissement cutané, ainsi que des supports et excipients acceptables en dermatologie et en cosmétologie.

Ainsi, les études réalisées par la demanderesse ont démontré de manière inattendue que l'association d'extraits d'anchuse, de pétales ou de mucilage de pétales de coquelicot et de passiflore présente un effet vérifié sur des kératinocytes humains en culture, se traduisant par un effet protecteur et réparateur de l'ADN mitochondrial nettement supérieur à celui de chacun de ses composants. Il est intéressant de noter que l'association de passiflore et d'anchuse décrite dans la demande WO 2009147345 précitée, a essentiellement des effets contre les contractions musculaires, en particulier les contractions faciales et les rides d'expression, tandis que les propriétés de l'association suivant la présente invention permettent de l'utiliser plus généralement pour la prévention et/ou le traitement des signes du vieillissement cutané.

De plus, aux doses utilisées, les essais effectués ont montré que les extraits utilisés dans la présente invention ne présentent aucune cytotoxicité.

L'extrait de coquelicot utilisé dans l'invention est un extrait de pétales de coquelicot, de préférence un extrait obtenu à partir du mucilage, ce qui évite les pigments et les opioïdes. Il faut noter que les propriétés des extraits de graines de coquelicot (Papaver rhoeas) se différencient sensiblement de celles des pétales, et plus particulièrement du mucilage. On sait en effet que les graines contiennent principalement des protéines et de la rhoeadine tandis que les pétales, et en particulier le mucilage, contiennent des polysaccharides.

Suivant une méthode préférée, les pétales sont extraits par l'eau en pH acide (4,6 - 6,6), l'extraction étant suivie d'une décoloration. L'extrait aqueux ainsi obtenu peut être conservé en présence d'un conservateur autorisé pour les produits biologiques comme le benzoate de soude et le sorbate de potassium.

L'extrait de mucilage de pétales de coquelicot se présente sous la forme d'un liquide se caractérisant par :
- matières sèches : 0,5 à 2%
- densité : 00,980 à 1,020
- indice de réfraction (à 22°C) : 01,320 à 1,350
- teneur en oses neutres : 015 à 45% (moyenne 40%)
- pH (en prise directe) : 04,6 à 6, 6 (moyenne 5).

Les matières sèches peuvent représenter de 0,5 à 2% comme indiqué ci-dessus, mais plus souvent 1,5 à 2%. La teneur en oses neutres est exprimée par rapport à la matière sèche.

L'extrait de pétales de coquelicot utilisé dans la présente invention et décrit ci-dessus peut être concentré et lyophilisé, si nécessaire.

En ce qui concerne l'anchuse et la passiflore, on utilise de préférence les parties aériennes, en particulier les sommités fleuries, comprenant les fleurs et jeunes feuilles de la partie apicale, de préférence aux autres parties des plantes.

Les extraits d'anchuse et de passiflore sont préparés à partir des plantes séchées, l'extrait représentant environ 5% du poids total de la plante. On peut par exemple préparer des extraits hydro-alcooliques, par exemples des extraits hydroglycoliques ou hydroéthanoliques, ou des extraits hydroglycérinés comme indiqué ci-après. Après séchage et pulvérisation des plantes, l'extraction se fait de préférence par percolation à raison de 100 g de plante pour 500 g d'eau. Le gâteau est exprimé et les liqueurs réunies et complétées à 500 g par addition de glycérine biologique.

L'extrait de passiflore utilisé dans l'invention présente les caractéristiques suivantes. Dans cet exemple, l'extrait utilisé est un extrait hydroglycériné obtenu à partir de l'espèce Passiflora incarnata.

Passiflora incarnata (extrait sec) (parties aériennes fleuries) 0,5 % :
- Eau : 049,75 %
- Glycérine : 049,75 %
- Densité à 22°C (1,050 - 1,150) : 01,117
- Indice de réfraction à 22°C (1,380 - 1,420) : 01,400
- pH (4, 9 à 6,9) : 05,89

Les caractéristiques de l'extrait d'anchuse utilisé dans l'invention sont indiquées ci-après. L'espèce d'anchuse utilisée dans cet exemple est Anchusa arvensis.

Anchusa arvensis (extrait sec) (sommités fleuries) 1, 0 % :
- Eau : 049,5 %
- Glycérine : 049,5 %
- Densité à 22°C (1,050 - 1,150) : 01,122
- Indice de réfraction à 22°C (1,380 - 1,420) : 01,401
- pH (6,0 à 8,0) : 07,0

Les extraits hydroglycérinés utilisés dans l'invention présentent l'avantage de pouvoir être conservés pendant une période prolongée dans des conditions normales de température et d'humidité sans qu'il soit nécessaire d'ajouter un conservateur. Il est cependant possible de diminuer la quantité de glycérine et d'ajouter un conservateur usuel. Ainsi, on peut utiliser un extrait à 20 ou 30% de glycérine additionné d'un conservateur approprié tel que le benzoate de sodium ou le sorbate de potassium. Suivant une variante de l'invention, on peut préparer un extrait hydroalcoolique (par exemple avec du butylène glycol ou du propylène glycol) complété par un conservateur usuel. Un tel extrait présente l'avantage de permettre une concentration un peu plus élevée en principe actif.

Parmi les diverses espèces du genre passiflore utilisables dans l'invention, on peut citer en particulier Passiflora incarnata, Passiflora acuminata, Passiflora alata, Passiflora antioquensis, Passiflora antiquiensis, Passiflora aurantia, Passiflora baraquiniana, Passiflora biflora, Passiflora caerulea, Passiflora capsularis, Passiflora coccinea, Passiflora cumbalensis, Passiflora dictamo, Passiflora edulis, Passiflora glandulosa, Passiflora hastata, Passiflora hircina, Passiflora laurifolia, Passiflora ligularis, Passiflora mexicana, Passiflora mixta, Passiflora mollissima, Passiflora morifolia, Passiflora multiflora, Passiflora nitida, Passiflora ornithoura, Passiflora quadrangularis, Passiflora saponaria, Passiflora suberosa, Passiflora sulcata, Passiflora tenuiloba, Passiflora tinifolia, Passiflora vitifolia, Passiflora foetida, Passiflora nigelliflora, Passiflora obscura, Passiflora gossypifolia, Passiflora grandiflora, Passiflora hastada, Passiflora hibisciflora, Passiflora hirsina, Passiflora hirsuta, Passiflora hispida. Suivant l'invention on utilise de préférence un extrait de l'espèce Passiflora incarnata.

Les espèces du genre anchuse utilisables dans les compositions de l'invention peuvent être choisies parmi Anchusa arvensis, Anchusa azurea, Anchusa barrelieri, Anchusa crispa, Anchusa gmelini, Anchusa italica, Anchusa lutea, Anchusa officinalis, Anchusa ovata, Anchusa paniculata, Anchusa sempervirens, Anchusa stricosa, Anchusa sylvestris, Anchusa tinctoria, Anchusa undulata / hybrida. Suivant l'invention, on utilise de préférence un extrait de l'espèce Anchusa arvensis ou sylvestris.

L'espèce de coquelicot utilisée de préférence dans l'invention est Papaver rhoeas, mais il est possible d'utiliser des extraits de pétales, et le cas échéant de mucilage, d'autres espèces telles que par exemple Papaver somniferum var. Nigrum (pavot oeillette), Papaver alpinum, Papaver argemone, Papaver collinum, Papaver dubium, Papaver hybridum, Papaver lamottei, Papaver modestum, Papaver pallidum, Papaver pinnatifidum, Papaver uniflorum.

Les tests d'évaluation de l'effet sur l'ADN mitochondrial ont été effectués sur des kératinocytes humains en culture, en utilisant des échantillons de chacun des composants isolément et de leur association.

Les résultats des tests in vitro détaillés ci-après, ont mis en évidence l'effet protecteur et régénérateur de l'ADN mitochondrial sans effet secondaire néfaste, et en particulier :
- l'absence de lésion d'ADN mitochondrial induite par l'association de l'invention aux doses testées ;
- une réduction significative des lésions provoquées par l'exposition des kératinocytes aux rayons ultraviolets en utilisant l'association de l'invention avant exposition, démontrant l'effet protecteur ;
- une réduction significative des lésions provoquées par l'exposition des kératinocytes aux rayons ultraviolets en utilisant l'association de l'invention après exposition, démontrant l'effet régénérateur ;
- l'absence de cytotoxicité de l'association des extraits de l'invention.

Ainsi les résultats des essais réalisés montrent que non seulement la composition de l'invention procure un effet protecteur et un effet réparateur de l'ADN mitochondrial de cellules exposées aux rayons UV, mais aussi que ces effets sont supérieurs à ceux des composants utilisés isolément dans les mêmes conditions, c'est-à-dire que la composition de l'invention procure un effet synergique inattendu.

On sait que sous l'action d'agressions physico-chimiques ou physio-pathologiques, les cellules humaines tentent de se protéger par activation de la transcription de gènes codant pour leurs protéines vitales, mais ces agressions peuvent cependant réduire cette capacité de moduler la transcription, ce qui peut priver les cellules des activités vitales telles que les fonctions mitochondriales et aboutir à la mort cellulaire par apoptose. L'exposition à des rayons UV, notamment les UVB, peut produire de tels effets néfastes. Ainsi, l'exposition à des doses importantes d'UV peut provoquer la paralysie mitochondriale et l'apparition de signes précoces d'apoptose. Les essais d'exposition de kératinocytes, c'est-à-dire les principales cellules de la peau humaine, sont donc particulièrement significatifs de la capacité de la composition de l'invention à protéger et réparer l'ADN mitochondrial.

Les résultats sont explicités dans les exemples ci-après.

La mitochondrie est un organite présent à l'intérieur de la cellule eucaryote qui joue un rôle physiologique primordial dans la synthèse des protéines, et donc dans le maintien de la structure de la peau. Elle est responsable des dernières étapes du cycle respiratoire pour produire l'énergie directement utilisable par la cellule (ATP) et gère l'assimilation des lipides et protéines apportés par la nutrition. La mitochondrie permet aussi la synthèse de collagène, d'acide hyaluronique, de céramides, et protège contre l'oxydation. Le maintien de la mitochondrie en état de bon fonctionnement est donc essentiel dans la conservation des qualités de la peau.

Il en résulte que l'effet de protection de l'ADN mitochondrial a des conséquences directes sur la synthèse des collagènes et de l'élastine, la synthèse des GAGs (glycosaminoglycanes) et des protéoglycanes, l'activité antiradicalaire, l'assimilation des glucides et plus généralement la limitation de la formation de rides, le raffermissement de la peau et la préservation de l'éclat du teint.

La composition selon l'invention peut comprendre par exemple entre 0,2 et 2% en poids d'extrait d'anchuse, entre 0,5 et 5% en poids d'extrait de pétales de coquelicot, et entre 0,5 et 6% en poids d'extrait de passiflore, par rapport au poids total de la composition.

Le choix de la concentration en chacun des composants dans la composition peut être fait en fonction de l'utilisation envisagée. Pour un traitement prolongé, on utilise de préférence des doses plus faibles, sous forme de lait ou de crème dosée à environ 2 à 8% (total des trois composants), tandis qu'un traitement ponctuel peut nécessiter des doses plus élevées, par exemple un gel ou un sérum dosé entre 8 et 15%.

Ainsi, ces compositions topiques peuvent être utilisées avantageusement en dermatologie et en cosmétologie pour le traitement ou la prévention des signes du vieillissement cutané.

Les compositions selon l'invention peuvent contenir, outre les extraits d'anchuse, de pétales de coquelicot, et de passiflore, des actifs secondaires renforçant ou complétant avantageusement leur activité, et compatibles, c'est-à-dire non susceptibles de réagir les uns sur les autres ou de masquer ou limiter leurs effets respectifs.

Plus particulièrement, les actifs secondaires peuvent être choisis parmi un agent anti-âge complémentaire, ou parmi des polyphénols de cacao, un extrait de graines de mimosa et un extrait de graines de souci agissant favorablement sur la stimulation des collagènes néoformés, ou encore un agent restructurant tel que Centella asiatica qui a un effet raffermissant et stimulant de la microcirculation cutanée.

L'agent anti-âge complémentaire peut être par exemple un lipide tel que le géranyl géranyl propanol (Juvinyl^{®}) qui réduit la formation des peroxydes intracellulaires et retarde ainsi le vieillissement cellulaire,

L'association d'extraits de pétales de coquelicot, d'anchuse et de passiflore suivant la présente invention, complétée le cas échéant par des substances à activité complémentaire, comme indiqué ci-dessus, utilisée dans des conditions normales d'emploi dans le traitement des signes du vieillissement cutané, pendant une période de 30 à 90 jours consécutifs, a montré une excellente tolérance cutanée.

Les extraits de pétales de coquelicot, d'anchuse et de passiflore de l'invention sont plus particulièrement destinés à des compositions pour administration par voie topique externe, c'est-à-dire une administration pour une action localisée directe, non systémique, sans passage par le système sanguin, à la différence d'une administration orale.

Les compositions conformes à la présente invention peuvent être présentées sous les formes galéniques classiquement utilisées pour une application topique dans cette indication, c'est-à-dire sous forme de gel, émulsion (en particulier crème ou lait), masque, pommade, lotion, solution concentrée, nanocapsules, ou liposomes, contenant des excipients et supports usuels compatibles et pharmaceutiquement acceptables. Elles sont utilisées de préférence sous forme de crèmes, de sérums, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on peut utiliser des émulsions à structure lamellaire obtenues avec des lécithines hydrogénées ou des sucro-esters, contenant peu de produits éthoxylés ou n'en contenant pas du tout.

Dans le cas des nanocapsules et des liposomes, il peut être avantageux d'utiliser des extraits aqueux de préférence à des extraits hydroglycoliques.

Suivant une forme de réalisation de l'invention, les extraits d'anchuse, de pétales de coquelicot et de passiflore peuvent être utilisés avantageusement sous forme encapsulée dans des liposomes. Suivant une technique connue dans la fabrication des compositions cosmétiques, les liposomes sont constitués par des petites sphères creuses, de diamètre généralement inférieur à 500 nm, dont la paroi est formée d'une double couche de lipides tels que des glucolipides ou des phospholipides, c'est-à-dire de nature proche de celle de la membrane cellulaire, facilitant la pénétration dans la peau. Ils peuvent être obtenus par exemple par traitement aux ultrasons d'un mélange d'un soluté aqueux et de lipides. Les lipides (phospholipides ou glucolipides) se réorganisent dans une configuration où l'énergie de l'ensemble est minimale, donc thermodynamiquement la plus stable. Les liposomes sont utilisés dans l'industrie cosmétique pour délivrer des composés à l'intérieur des cellules lorsque le vésicule fusionne avec la membrane plasmique.

Les compositions topiques selon l'invention peuvent comprendre des excipients appropriés pour une administration topique externe, en particulier des excipients acceptables sur le plan dermatologique et cosmétologique. Ces excipients appropriés pour la formulation sont bien connus de l'homme du métier et comprennent en particulier des agents de pénétration tels que l'éthoxydiglycol, le phytantriol, l'octyl dodécanol et l'escine ; les épaississants tels que les gommes naturelles et les polymères de synthèse ; les émollients et les tensioactifs tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol, le parahydroxybenzoate de méthyle (méthylparaben), le parahydroxybenzoate d'éthyle (éthylparaben) et le Phenonip® associant du phénoxyéthanol et des parahydroxybenzoates ; les colorants ; les parfums ; etc.

Comme autres ingrédients utilisables dans les compositions de l'invention, on peut citer les agents hydratants tels que le propylène glycol, la glycérine, le butylène glycol, le sel de sodium de l'acide pyrrolidone carboxylique (sodium PCA), ainsi des associations de dérivés glucosiques à effet hydratant et restructurant comme le produit Aquaxyl® (xylityl-glucoside et anhydroxylytol et xylitol), et également les vitamines antioxydantes telles que la vitamine E, par exemple l'acétate de tocophérol ou le tocotriénol, la vitamine C, les polyphénols naturels. On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane. Les pigments peuvent être choisis par exemple parmi le dioxyde de titane, l'oxyde de zinc, l'oxyde de zirconium ou encore l'oxyde d'aluminium.

Les exemples suivants illustrent l'invention plus en détail sans en limiter la portée. Dans tous les exemples de compositions qui suivent, les parties sont exprimées en poids, sauf indication contraire.

### Exemple 1

Suivant les techniques classiques, on prépare un sérum anti-âge ayant la composition pondérale suivante.

| | |
|---|---|
| Eau déminéralisée | qsp 100,00 |
| Tetrasodium glutamate diacétate | 0,07 |
| Butylène glycol végétal | 5,00 |
| Gomme xanthane | 0,05 |
| Hydroxyéthyl cellulose | 0,30 |
| Lécithine hydrogénée | 1,00 |
| Bentonite | 1,00 |
| Caprylique/caprique triglycérides | 3,00 |
| Stéaroyl lactylate de sodium | 0,20 |
| Geranylgeranyl propanol | 2,00 |
| Undécylénate de glycéryle | 0,50 |
| Tetra-isopalmitate d'ascorbyle | 0,20 |
| Acide dehydroacétique, sel de sodium | 0,10 |
| Hyaluronate de sodium | 0,15 |
| Tréhalose | 1,00 |
| Complexe suivant l'invention | 10,00 |
| Xylityl glucoside et anhydro-xylitol et Aqua et xylitol (Aquaxyl^{®}) | 1,00 |
| Amidon de maïs | 0,50 |

Ce sérum est préparé suivant les techniques usuelles.

Le complexe suivant l'invention associe les extraits d'anchuse, de coquelicot (pétales) et de passiflore aux teneurs respectives de 2,00%, 3,75% et 4,25%.

Le sérum ayant la composition indiquée ci-dessus peut être utilisé en application locale sur le visage, une à deux fois par jour pendant 6 à 12 semaines, ce traitement pouvant être répété plusieurs fois par an. Les premiers effets de diminution des rides sont observés dès la 4^{ème} semaine.

### Exemple 2

Suivant une technique usuelle, on prépare une crème régénératrice nourrissante ayant la composition pondérale suivante.

| | |
|---|---|
| Eau déminéralisée | qsp 100,00 |
| Tetrasodium glutamate diacétate | 0,07 |
| Butylène glycol végétal | 5,00 |
| Glycérine bio | 8,00 |
| Bétaïne | 2,00 |
| Hydroxyéthyl cellulose | 0,50 |
| Dehydroacétate de sodium | 0,15 |
| Alcools C₁₂-C₁₆, acide palmitique, lécithine hydrogénée (Biophilic H^{®}) | 4,00 |
| Glyceryl stearate citrate | 0,80 |
| Squalane végétal | 5,00 |
| Caprylique/caprique triglycérides | 7,00 |
| Alcool béhénylique | 1,00 |
| Huile de Macadamia | 2,00 |
| Huile de Jojoba | 0,50 |
| Beurre de Karité | 4,00 |
| Silicate de magnésium | 1,00 |
| Undécylénate de glycéryle | 0,50 |
| Tetra-isopalmitate d'ascorbyle | 0,30 |
| Complexe suivant l'invention | 8,00 |
| Tréhalose | 1,00 |
| Hyaluronate de sodium | 0,20 |
| Matrixyl 3000 | 3,00 |
| Parfums | 0,50 |

Le complexe suivant l'invention associe les extraits d'anchuse, de coquelicot (pétales) et de passiflore aux teneurs respectives de 1,6%, 3,0% et 3,4%.

La crème régénératrice et nourrissante ayant la composition indiquée ci-dessus peut être utilisée en application sur les zones à traiter, une fois par jour pendant une période de 1 à 3 mois.

### Exemple 3

Suivant les techniques classiques, on prépare une lotion rafraîchissante ayant la composition pondérale suivante.

| | |
|---|---|
| Eau déminéralisée | qsp 100,00 |
| Butylène glycol végétal | 5,00 |
| Acide galactarique | 0,10 |
| Acide anisique | 0,10 |
| Acide levulinique / levulinate de sodium | 0,30 |
| Eau de menthe poivrée | 10,00 |
| Eau de Melisse | 10,00 |
| Eau de tilleul | 10,00 |
| Eau d'Hamamelis | 25,00 |
| Extrait glycolique de Centella asiatica | 5,00 |
| Eau de Romarin | 3,00 |
| Complexe suivant l'invention | 2,00 |

Le complexe suivant l'invention associe les extraits d'anchuse, de coquelicot (pétales) et de passiflore aux teneurs respectives de 0,40%, 0,75% et 0,85%.

Cette lotion ayant la composition indiquée ci-dessus, et préparée suivant les techniques usuelles, est utilisée en application locale sur le visage, une à deux fois par jour pendant 6 à 10 semaines.

### Exemple 4

L'étude in vitro de l'effet de l'association utilisée dans la présente invention sur la protection et la réparation de l'ADN mitochondrial a été faite sur des kératinocytes humains en culture.

### Kératinocytes humains en culture :

Les kératinocytes ont été isolés à partir d'échantillons prélevés au cours d'opérations chirurgicales de manière usuelle, et ont été cultivés jusqu'à obtention du nombre de cellules nécessaires à l'étude. L'étude a été réalisée sur des cellules cultivées dans des plaques 6 puits à raison de 2x10⁶ cellules par puits dans du milieu de culture supplémenté en extrait de glandes pituitaires bovines, EGF recombinant humain, insuline, hydrocortisone, transférine humaine, épinéphrine et chlorure de calcium.

L'essai de cytotoxicité a été réalisé en triplicate après 24 heures de contact avec les produits testés.

Les lots suivants ont été constitués.
Lot n°1 : témoin ne recevant aucun produit.
Lot n°2 : traité par le Complexe 1.
Lot n°3 : traité par le Complexe 2.
Lot n°4 : traité par le Complexe 3.
Lot n°5 : traité par l'extrait d'anchuse 0,4%.
Lot n°6 : traité par l'extrait d'anchuse 0,8%.
Lot n°7 : traité par l'extrait d'anchuse 1,6%.
Lot n°8 : traité par l'extrait de coquelicot 0,75%.
Lot n°9 : traité par l'extrait de coquelicot 1,5%.
Lot n°10 : traité par l'extrait de coquelicot 3,0%.
Lot n°11 : traité par l'extrait de passiflore 0,85%.
Lot n°12 : traité par l'extrait de passiflore 1,7%.
Lot n°13 : traité par l'extrait de passiflore 3,4%.
Complexe n°1 : extrait d'anchuse 0,4% + extrait de pétales de coquelicot 0,75% + extrait de passiflore 0,85%.
Complexe n°2 : extrait d'anchuse 0,8% + extrait de pétales de coquelicot 1,5% + extrait de passiflore 1,7%.
Complexe n°3 : extrait d'anchuse 1,6% + extrait de pétales de coquelicot 3,0% + extrait de passiflore 3,4%.

La viabilité cellulaire a été déterminée par le test de réduction au bleu de Formazan (test MTT).

Après 24 heures d'incubation des cellules de kératinocytes de chaque lot où les produits à tester ont été appliqués à différentes concentrations en comparaison avec le témoin comme indiqué ci-dessus, les puits contenant les cellules sont vidés par retournement doux et le tapis cellulaire est rincé avec le milieu de culture. On distribue dans tous les puits 200 µl d'une solution diluée de MTT (bromure de 3-(4,5-diméthylthiazol-2-yl)-2,5-diphényltétrazolium), puis les plaques sont incubées à 37°C pendant 3 heures. On observe alors la formation de cristaux bleu de Formazan en quantité inversement proportionnelle à l'atteinte des succinates déshydrogénases

Les puits sont à nouveau vidés par retournement, les cellules sont ensuite lysées et les cristaux de bleu de Formazan sont dissous par 200 µl de diméthylsulfoxyde. Après homogénéisation de la coloration par agitation, la densité optique (DO) des plaques est lue, au moyen d'un spectrophotomètre à 570 nm, ce qui permet de connaître la quantité relative de cellules vivantes et actives métaboliquement.

Les valeurs de la densité optique (DO) après 24 heures de contact sont regroupées dans le tableau ci-dessous.

| Substance | Densité optique DO 570 nm | % |
|---|---|---|
| Témoin | 0,580 ± 0,02 | - |
| Complexe n°1 | 0,590 ± 0,05 | ns |
| Complexe n°2 | 0,584 ± 0,03 | ns |
| Complexe n°3 | 0,592 ± 0,06 | ns |
| Extrait d'anchuse 0,4% | 0,575 ± 0,03 | ns |
| Extrait d'anchuse 0,8% | 0,592 ± 0,02 | ns |
| Extrait d'anchuse 1,6% | 0,586 ± 0,04 | ns |
| Extrait de coquelicot 0,75% | 0,570 ± 0,04 | ns |
| Extrait de coquelicot 1,5% | 0,587 ± 0,03 | ns |
| Extrait de coquelicot 3,0% | 0,574 ± 0,05 | ns |
| Extrait de passiflore 0,85% | 0,590 ± 0,04 | ns |
| Extrait de passiflore 1,7% | 0,584 ± 0,06 | ns |
| Extrait de passiflore 3,4% | 0,595 ± 0,05 | ns |

Ces résultats montrent que les extraits utilisés dans l'invention, ainsi que leur association, ne présentent aucune cytotoxicité vis-à-vis des kératinocytes humains en culture aux concentrations étudiées.

### Exemple 5

L'étude de l'effet des extraits utilisés dans l'invention et de leur association sur l'ADN mitochondrial a été effectuée comme indiqué ci-après.

On utilise les mêmes kératinocytes en culture que dans l'Exemple 4. Les essais sont effectués dans des conditions physiologiques, puis dans des conditions d'induction de dégradations par irradiation aux rayons UVB, soit après application des produits pour évaluer leur effet protecteur, soit avant application des produits pour évaluer leur effet réparateur. Chacun de ces trois essais a été réalisé en triplicate pendant 24 heures.

Pour étudier l'effet des produits sur l'ADN mitochondrial, après 24 heures de contact avec les produits, les cellules sont récupérées après 24 heures d'incubation avant extraction de l'ADN.

L'ADN total a été extrait à l'aide d'un kit d'extraction Invisorb^{®} Spin DNA Extraction Kit (Eurobio, France).

Les cellules sont lysées à l'aide d'un tampon de lyse et les acides nucléiques sont précipités en présence d'éthanol à 70%. L'ADN total est ensuite fixé directement sur la membrane d'une colonne dédiée à l'extraction des ADN. Après lavage de la membrane, l'ADN est élué au moyen d'un tampon à faible teneur en sel. L'ADN mitochondrial ne représente que 0,1% environ de l'ADN total. Il est donc amplifié au moyen d'un kit REPLI-g Mitochondrial DNA (Qiagen, France) contenant la DNA polymérase, les tampons et les réactifs nécessaires à l'amplification du génome mitochondrial humain à partir du génome entier avec un rendement approximatif de 3 à 5 µg d'ADN mitochondrial amplifié par réaction. L'ADN total est dénaturé à 75°C pendant 5 minutes par un tampon spécifique. Après arrêt de la dénaturation par refroidissement de la solution à température ambiante, la DNA polymérase est ajoutée. L'amplification isothermique a lieu pendant 8 heures à 33°C, puis l'ADN mitochondrial est congelé à -20°C.

La quantification des dommages causés à l'ADN mitochondrial est effectuée comme suit, en utilisant un DNA Damage Quantification Kit (Biovision, USA) utilisant des réactifs ARP (Aldéhyde Reactive Probe).

L'ADN mitochondrial (0,5 µg) est incubé avec les réactifs ARP pendant 1 heure à 37°C pour marquer les sites AP (apurinique/apyrimidinique), qui sont une des lésions principales de l'ADN et constituent un bon indicateur des lésions de l'ADN et du taux de réparation. L'ADN est ensuite précipité par l'éthanol pur puis centrifugé et lavé 3 fois à l'éthanol à 70%. Le culot d'ADN est séché à l'air libre puis repris par 1 ml de tampon TE. L'ADN marqué est appliqué sur une plaque 96 puits pendant une nuit, puis après 5 lavages, une solution HRP-streptavidine est ajoutée dans les puits pendant 1 heure à température ambiante. Après 5 lavages, le développement de la couleur est révélé par un tampon spécifique pendant 1 heure à 37°C puis stoppé par de l'acide sulfurique 2N. La densité optique est lue sur un spectrophotomètre à 450 nm pour quantifier les sites AP marqués.

Les résultats sont regroupés dans le tableau ci-après.

| Substance | Nb de sites AP/10⁵ paires de bases | % |
|---|---|---|
| Témoin | 0,64 ± 0,04 | - |
| Complexe n°1 | 0,60 ± 0,02 | ns |
| Complexe n°2 | 0,63 ± 0,04 | ns |
| Complexe n°3 | 0,59 ± 0,05 | ns |
| Extrait d'anchuse 0,4% | 0,62 ± 0,03 | ns |
| Extrait d'anchuse 0,8% | 0,68 ± 0,025 | ns |
| Extrait d'anchuse 1,6% | 0,62 ± 0,037 | ns |
| Extrait de coquelicot 0,75% | 0,65 ± 0,05 | ns |
| Extrait de coquelicot 1,5% | 0,67 ± 0,02 | ns |
| Extrait de coquelicot 3,0% | 0,65 ± 0,04 | ns |
| Extrait de passiflore 0,85% | 0,65 ± 0,05 | ns |
| Extrait de passiflore 1,7% | 0,61 ± 0,07 | ns |
| Extrait de passiflore 3,4% | 0,63 ± 0,06 | ns |

Ces résultats montrent que les extraits étudiés ainsi que l'association de l'invention, aux concentrations testées, n'ont induit aucune lésion d'ADN mitochondrial dans les conditions physiologiques.

Comme indiqué plus haut, l'effet protecteur des produits a été évalué vis-à-vis des dommages causés à l'ADN mitochondrial par les UVB au niveau des kératinocytes humains en culture.

A cet effet, les cellules sont traitées par les produits à tester, à différentes concentrations, pendant 2,5 heures, puis irradiées aux rayons UVB (100 mJ/cm²). Elles sont récupérées après 24 heures d'incubation.

Les résultats sont regroupés dans le tableau ci-après.

| Substance | Nb de sites AP/10⁵ paires de bases | % |
|---|---|---|
| Témoin négatif | 0,64 ± 0,04 | - |
| Témoin + UVB | 1,66 ± 0,06 | +159 |
| Complexe n°1 + UVB | 1,31 ± 0,06 | -21 |
| Complexe n°2 + UVB | 1,23 ± 0,07 | -26 |
| Complexe n°3 + UVB | 1,19 ± 0,09 | -28 |
| Extrait d'anchuse 0,4% + UVB | 1,46 ± 0,04 | -12 |
| Extrait d'anchuse 0,8% + UVB | 1,44 ± 0,05 | -13 |
| Extrait d'anchuse 1,6% + UVB | 1,46 ± 0,07 | -12 |
| Extrait de coquelicot 0,75% + UVB | 1,40 ± 0,07 | -16 |
| Extrait de coquelicot 1,5% + UVB | 1,37 ± 0,03 | -17 |
| Extrait de coquelicot 3,0% + UVB | 1,32 ± 0,08 | -20 |
| Extrait de passiflore 0,85% + UVB | 1,42 ± 0,06 | -14 |
| Extrait de passiflore 1,7% + UVB | 1,36 ± 0,02 | -18 |
| Extrait de passiflore 3,4% + UVB | 1,38 ± 0,04 | -17 |

Ces résultats montrent que le nombre de sites AP est stimulé en réaction aux rayons UVB (+159%) par comparaison avec le témoin négatif. Le traitement des cellules de kératinocytes par les produits testés avant exposition aux UVB réduit significativement les lésions de l'ADN mitochondrial dans le cas du traitement par l'association de l'invention tandis que l'effet protecteur est sensiblement moins important avec chacun des composants séparément.

L'effet réparateur des produits a été évalué vis-à-vis des dommages causés à l'ADN mitochondrial par les UVB au niveau des kératinocytes humains en culture.

A cet effet, les cellules sont irradiées aux rayons UVB (100 mJ/cm²), puis incubées pendant 3 heures. Les produits à tester sont ensuite ajoutés aux cultures cellulaires irradiées, à différentes concentrations. Les cultures sont récupérées après 24 heures d'incubation.

Les résultats sont regroupés dans le tableau ci-après.

| Substance | Nb de sites AP/10⁵ paires de bases | % |
|---|---|---|
| Témoin négatif | 0,64 ± 0,04 | - |
| UVB + Témoin | 1,71 ± 0,04 | +167 |
| UVB + Complexe n°1 | 1,28 ± 0,07 | -25 |
| UVB + Complexe n°2 | 1,16 ± 0,09 | -32 |
| UVB + Complexe n°3 | 0,98 ± 0,10 | -43 |
| UVB + Extrait d'anchuse 0,4% | 1,51 ± 0,08 | -12 |
| UVB + Extrait d'anchuse 0,8% | 1,40 ± 0,06 | -28 |
| UVB + Extrait d'anchuse 1,6% | 1,33 ± 0,04 | -22 |
| UVB + Extrait de coquelicot 0,75% | 1,42 ± 0,05 | -17 |
| UVB + Extrait de coquelicot 1,5% | 1,34 ± 0,09 | -22 |
| UVB + Extrait de coquelicot 3,0% | 1,29 ± 0,07 | -25 |
| UVB + Extrait de passiflore 0,85% | 1,41 ± 0,06 | -18 |
| UVB + Extrait de passiflore 1,7% | 1,37 ± 0,02 | -20 |
| UVB + Extrait de passiflore 3,4% | 1,34 ± 0,04 | -22 |

Ces résultats montrent que le nombre de sites AP est stimulé en réaction aux rayons UVB (+167%) par comparaison avec le témoin négatif. Le traitement des cellules de kératinocytes par les produits testés après exposition aux UVB réduit significativement les lésions de l'ADN mitochondrial dans le cas du traitement par l'association de l'invention tandis que l'effet réparateur est sensiblement moins important avec chacun des composants séparément.

Cet effet protecteur et réparateur vis-à-vis des UVB, et l'effet de synergie de l'association des trois composants de la composition de l'invention (extraits de sommités fleuries d'anchuse, de sommités fleuries de passiflore, et de pétales de coquelicot), ont été confirmés par analyse de la transcription d'une batterie de 84 gènes (gènes codant pour la survie ou l'apoptose de cellules humaines). Pour cette analyse, les cellules sont irradiées soit avant addition des divers extraits (effet réparateur), soit après (effet protecteur), et après irradiation les RNAm cellulaires sont purifiés, rétrotranscrits en ADNc, puis amplifiés par PCR quantitative, et les résultats sont comparés pour évaluer l'effet des extraits.

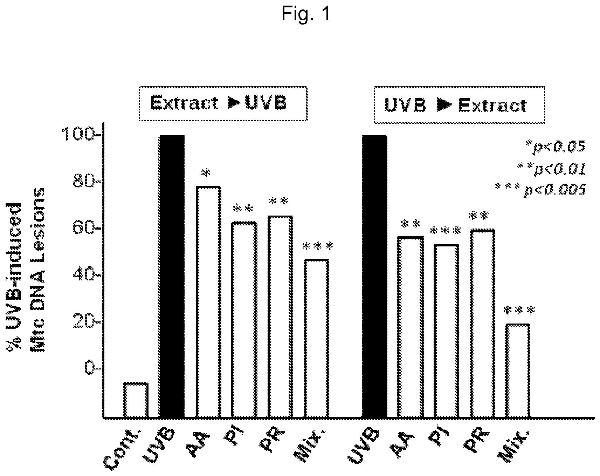

Les extraits utilisés proviennent de Passiflora incarnata (PI), Papaver rhoeas (PR) et Anchusa arvensis (AA) isolément, ou en mélange (MIX) suivant l'invention. Les résultats sont représentés sur la Fig. 1 qui met en évidence l'effet de synergie observé avec le mélange (MIX).

La meilleures protection contre les UVB est obtenue avec des taux de dilution de 0,8% pour AA, 3% pour PR et 1,7% pour PI avant irradiation.

De manière étonnante, quand les trois extraits sont mélangés (MIX), avant addition à des kératinocytes irradiés, un plateau est atteint avec des concentrations significativement inférieures de ces extraits. La meilleure protection est obtenue avec des cellules traitées par un mélange de 0,016% AA, 0,034% PI et 0,03% PR. De plus, le mélange procure une meilleure inhibition des dommages induits par les UVB par comparaison avec les valeurs optimales de chaque extrait isolément, ce qui met en évidence un effet de synergie dans le mélange.

Dans le test sur les effets réparateurs (2^{ème} partie de la Fig. 1) la réversion optimale des dommages induits par les UVB est observée à des taux de dilution de 1,6% pour AA, 3% pour PR et 3,4% pour PI, procurant des taux de réduction des lésions de mtcDNA de 36%, 39% et 35% respectivement. De manière étonnante, le taux de réduction est de 68% avec le mélange (MIX).

L'association d'anchuse, de pétales de coquelicot et de passiflore suivant l'invention joue donc un rôle à la fois protecteur et réparateur de l'ADN mitochondrial, ce qui permet d'en faire une composition efficace pour la protection de la peau.

## Revendications

1. Composition cosmétique et/ou dermatologique pour application topique, destinée à la protection de la peau, **caractérisée en ce qu'**elle comprend une quantité efficace d'extraits d'anchuse, de pétales ou de mucilage de pétales de coquelicot et de passiflore, en association.

2. Composition selon la revendication 1, **caractérisée en ce qu'**elle comprend une association d'extraits d'anchuse, de pétales ou de mucilage de pétales de coquelicot et de passiflore en une quantité efficace pour la prévention et/ou le traitement des signes du vieillissement cutané, ainsi que des supports et excipients acceptables en dermatologie et en cosmétologie.

3. Composition selon l'une quelconque des revendications 1 et 2, **caractérisée en ce que** l'extrait d'anchuse est un extrait de Anchusa arvensis ou Anchusa sylvestris.

4. Composition selon l'une quelconque des revendications 1 et 2, **caractérisée en ce que** l'extrait de coquelicot est un extrait de Papaver rhoeas.

5. Composition selon l'une quelconque des revendications 1 et 2, **caractérisée en ce que** l'extrait de passiflore est un extrait de Passiflora incarnata.

6. Composition selon la revendication 1, **caractérisée en ce qu'**elle comprend un extrait hydroglycériné d'anchuse ayant les caractéristiques suivantes :
- Eau : 049,5 %
- Glycérine : 049,5 %
- Densité à 22°C : 01,050 - 1,150
- Indice de réfraction à 22°C : 01,380 - 1,420
- pH : 06,0 à 8,0

7. Composition selon la revendication 1, **caractérisée en ce qu'**elle comprend un extrait de pétales de coquelicot ayant les caractéristiques suivantes :
- matières sèches 00,5 à 2%
- densité 00,980 à 1,020
- indice de réfraction (à 22°C) 01,320 à 1,350
- teneur en oses neutres 015 à 45% (moyenne 40%)
- pH (en prise directe) : 04,6 à 6,6 (moyenne 5).

8. Composition selon la revendication 1, **caractérisée en ce qu'**elle comprend un extrait hydroglycériné de passiflore ayant les caractéristiques suivantes :
- Eau : 049,75 %
- Glycérine : 049,75 %
- Densité à 22°C : 01,050 - 1,150
- Indice de réfraction à 22°C : 01,380 - 1,420
- pH : 04,9 à 6,9

9. Composition selon l'une quelconque des revendications 1 à 8, **caractérisée en ce qu'**elle comprend entre 0,2 et 2% en poids d'extrait d'anchuse, entre 0,5 et 5% en poids d'extrait de pétales de coquelicot, et entre 0,5 et 6% en poids d'extrait de passiflore, par rapport au poids total de la composition.

10. Composition comprenant des extraits d'anchuse, de pétales ou de mucilage de pétales de coquelicot et de passiflore, en association, pour utilisation dans la prévention et/ou le traitement des signes du vieillissement cutané.

## Patentansprüche

1. Kosmetische und/oder dermatologische Zusammensetzung zur topischen Anwendung, ausgelegt zum Schutz der Haut, **dadurch gekennzeichnet, dass** sie eine wirksame Menge von Extrakten aus Anchusa, Blumenblättern oder Schleim der Blumenblätter von Klatschmohn und von Passionsblume in Verbindung umfasst.

2. Zusammensetzung nach Anspruch 1, **dadurch gekennzeichnet, dass** sie eine Verbindung von Extrakten aus Anchusa, Blumenblättern oder Schleim der Blumenblätter von Klatschmohn und von Passionsblume in einer wirksamen Menge zur Prävention und/oder Behandlung der Zeichen der Alterung der Haut umfasst, ebenso wie dermatologisch und kosmetologisch annehmbare Träger und Trägerstoffe.

3. Zusammensetzung nach einem der Ansprüche 1 und 2, **dadurch gekennzeichnet, dass** der Extrakt aus Anchusa ein Extrakt aus *Anchusa arvensis* oder *Anchusa sylvestris* ist.

4. Zusammensetzung nach einem der Ansprüche 1 und 2, **dadurch gekennzeichnet, dass** der Extrakt aus Klatschmohn ein Extrakt aus *Papaver rhoeas* ist.

5. Zusammensetzung nach einem der Ansprüche 1 und 2, **dadurch gekennzeichnet, dass** der Extrakt aus Passionsblume ein Extrakt aus *Passiflora incarnata* ist.

6. Zusammensetzung nach Anspruch 1, **dadurch gekennzeichnet, dass** sie einen Hydroglycerin-Extrakt von Anchusa mit den folgenden Eigenschaften umfasst:
- Wasser: 049,5 %
- Glycin: 049,5 %
- Dichte bei 22 °C: 01,050 - 1,150
- Brechungsindex bei 22 °C: 01,380 - 1,420
- pH-Wert: 06,0 bis 8,0

7. Zusammensetzung nach Anspruch 1, **dadurch gekennzeichnet, dass** sie einen Extrakt aus Blumenblättern von Klatschmohn mit den folgenden Eigenschaften umfasst:
- Trockenmasse 00,5 bis 2 %
- Dichte 00,980 bis 1,020
- Brechungsindex (bei 22 °C) 01,320 bis 1,350
- Gehalt an neutralen Monosacchariden 15 bis 45 % (Mittelwert 40 %)
- pH (in direktem Kontakt) : 04,6 bis 6,6 (Mittelwert 5).

8. Zusammensetzung nach Anspruch 1, **dadurch gekennzeichnet, dass** einen sie Hydroglycerin-Extrakt aus Passionsblume mit den folgenden Eigenschaften umfasst:
- Wasser: 049,75 %
- Glycerin: 049,75 %
- Dichte bei 22 °C: 01,050 - 1,150
- Brechungsindex bei 22 °C: 01,380 - 1,420
- pH-Wert: 04,9 bis 6,9

9. Zusammensetzung nach einem beliebigen der Ansprüche 1 bis 8, **dadurch gekennzeichnet, dass** sie zwischen 0,2 und 2 Gew.% Extrakt aus Anchusa, zwischen 0,5 und 5 Gew.% Extrakt aus Blumenblättern von Klatschmohn und zwischen 0,5 und 6 Gew.% Extrakt aus Passionsblume mit Bezug auf das Gesamtgewicht der Zusammensetzung umfasst.

10. Zusammensetzung, umfassend Extrakte aus Anchusa, Blumenblättern oder Schleim von Blumenblättern von Klatschmohn und von Passionsblume in Verbindung zur Verwendung bei der Prävention und/oder der Behandlung von Zeichen der Alterung der Haut.

## Claims

1. Cosmetic and/or dermatological composition for topical application, intended to protect the skin, **characterized in that** it comprises an efficient amount of extracts of Anchusa, of poppy and Passiflora petals or petal mucilage, in association.

2. The composition according to claim 1, **characterized in that** it comprises an association of extracts of Anchusa, of poppy and Passiflora petals or petal mucilage in an efficient amount for the prevention and/or treatment of signs of skin ageing, and carriers and excipients acceptable in dermatology and cosmetology.

3. The composition according to any of claims 1 and 2, **characterized in that** the Anchusa extract is an extract of *Anchusa arvensis* or *Anchusa sylvestris.*

4. The composition according to any of claims 1 and 2, **characterized in that** the poppy extract is an extract of *Papaver rhoeas.*

5. The composition according to any of claims 1 and 2, **characterized in that** the Passiflora extract is an extract of *Passiflora incarnata.*

6. The composition according to claim 1, **characterized in that** it comprises a hydroglycerin extract of Anchusa having the following characteristics:
- Water. 049.5 %
- Glycerine: 049.5 %
- Density at 22°C: 01.050 - 1.150
- Refractive index at 22°C 01.380 - 1.420
- pH: 06.0 to 8.0

7. The composition according to claim 1, **characterized in that** it comprises an extract of poppy petals having the following characteristics:
- Dry matter: 00.5 to 2 %
- Density: 00.980 to 1.020
- Refractive index (at 22°C): 01.320 to 1.350
- Neutral ose content: 015 to 45 % (mean 40 %)
- pH (direct measurement): 04.6 to 6.6 (mean 5).

8. The composition according to claim 1, **characterized in that** it comprises a hydroglycerin extract of Passiflora having the following characteristics:
- Water: 049.75 %
- Glycerine: 049.75 %
- Density at 22°C 01.050 - 1.150
- Refractive index at 22 °C: 01.380 - 1.420
- pH: 04.9 to 6.9

9. The composition according to any of claims 1 to 8, **characterized in that** it comprises between 0.2 and 2 % by weight of Anchusa, between 0.5 and 5 % by weight of poppy petal extract, and between 0.5 and 6 % by weight of Passiflora extract, relative to the total weight of the composition.

10. Composition comprising extracts of Anchusa, of petals or petal mucilage of poppy and Passiflora, in association, for use in the prevention and/or treatment of signs of skin ageing.
